# EUROPÄISCHE PATENTSCHRIFT

(11) **EP 3 583 089 B1**
(45) Veröffentlichungstag und Bekanntmachung des Hinweises auf die Patenterteilung: **10.03.2021**
(21) Anmeldenummer: 18720182.7
(22) Anmeldetag: 18.04.2018
(51) Int. Cl.: C07C 29/151, C07C 31/04, B01J 8/00

(54) **VERFAHREN UND VORRICHTUNG ZUM UMSETZEN VON KOHLENSTOFFDIOXID ZU METHANOL**
METHOD AND DEVICE FOR CONVERTING CARBON DIOXIDE INTO METHANOL
PROCÉDÉ ET DISPOSITIF POUR LA TRANSFORMATION DE DIOXYDE DE CARBONE EN MÉTHANOL

(30) Priorität: 21.04.2017 DE 102017206763
(43) Veröffentlichungstag der Anmeldung: 25.12.2019
(73) Patentinhaber: Siemens Energy Global GmbH & Co. KG, 81739 München (DE)
(72) Erfinder: MELTZER, Katharina, 91052 Erlangen (DE); BALDAUF, Manfred, 91056 Erlangen (DE); TREMEL, Alexander, 91096 Möhrendorf (DE)
(86) Internationale Anmeldenummer: PCT/EP2018/059871
(87) Internationale Veröffentlichungsnummer: WO 2018/192957

(56) Entgegenhaltungen:
- EP-A1- 0 326 718
- WO-A1-2017/162513
- DE-A1-102015 215 662
- GB-A- 2 293 334

## Beschreibung

Die Erfindung betrifft ein Verfahren und eine Vorrichtung zum Umsetzen von Kohlenstoffdioxid und Wasserstoff zu Methanol und Wasser.

Die Nachfrage nach Strom schwankt im tageszeitlichen Verlauf stark. Auch die Stromerzeugung schwankt mit zunehmendem Anteil an Strom aus erneuerbaren Energien während des Tagesverlaufs. Um ein Überangebot an Strom in Zeiten mit viel Sonne und starkem Wind bei niedriger Nachfrage nach Strom ausgleichen zu können, benötigt man regelbare Kraftwerke oder Speicher, um diese Energie zu speichern.

Eine der derzeit angedachten Lösungen ist das Umwandeln von elektrischer Energie in Wertprodukte, die insbesondere als Plattformchemikalien dienen können. Eine mögliche Technik zur Umwandlung der elektrischen Energie in Wertprodukte stellt die Elektrolyse dar. Insbesondere die Elektrolyse von Wasser zu Wasserstoff und Sauerstoff stellt eine im Stand der Technik bekannte Methode dar.

Kohlenstoffdioxid ist ein klimaschädliches Treibhausgas. Es trägt maßgeblich zu dem Treibhauseffekt bei und somit zur globalen Klimaerwärmung. Die Verringerung des Ausstoßes von Kohlenstoffdioxid, insbesondere bei industriellen Prozessen, ist daher erwünscht. Um unterschiedliche industrielle Prozesse möglichst klimaneutral, d.h. mit geringem Kohlenstoffdioxidausstoß, zu betreiben, ist es wünschenswert, das Kohlenstoffdioxid, das bei diesen Prozessen entsteht, zu Wertstoffen umzuwandeln.

Der Wasserstoff, der in einer Elektrolyse hergestellt wurde, kann mit klimaschädlichem Kohlenstoffdioxid zu Methanol, einem Wertstoff, umgesetzt werden. Aufgrund der Reaktionsthermodynamik ist der Gleichgewichtsumsatz bei der Umsetzung von Kohlenstoffdioxid und Wasserstoff zu Methanol und Wasser stark limitiert.

Bei der konventionellen Methanolsynthese wird als Edukt Synthesegas, also ein Gemisch aus Wasserstoff, Kohlenstoffmonoxid und Kohlenstoffdioxid, verwendet. Es müssen harsche Reaktionsbedingungen von 50-100 bar und 200-300 °C vorliegen, um diese Reaktion durchführen zu können.

Wird anstelle des Synthesegases als Edukt überwiegend Kohlenstoffdioxid und Wasserstoff verwendet, werden um ein Vielfaches geringere Umsätze im Vergleich zur Herstellung von Methanol aus Synthesegas erreicht. Der Umsatz des Kohlenstoffdioxids bei diesen Reaktionsbedingungen liegt nachteiligerweise in einer niedrigen Größenordnung von etwa 20 %.

Um den Gesamtumsatz an Wasserstoff und Kohlenstoffdioxid zu Methanol zu vergrößern, werden das nicht reagierte Kohlenstoffdioxid und der nicht reagierte Wasserstoff im Kreis geführt. Da sich in den verwendeten Rohrleitungen und im Reaktor Druckverluste einstellen, ist ein Kompressor zur Rückverdichtung der rückgeführten und nicht reagierten Gase notwendig. Je größer die Menge an im Kreis geführten Gasgemisch ist, desto höher ist somit nachteiligerweise auch der Energiebedarf für die Umsetzung von Kohlenstoffdioxid zu Methanol, da der Kompressor eine große Energiemenge benötigt.

Die DE 10 2015 215 662 A1 beschreibt ein Verfahren zu Umsetzung von gleichgewichtslimitierten Reaktionen, umfassend folgende Schritte: Anordnen eines Katalysatormaterials in einem Reaktor, Einbringen von Edukten in den Reaktor, Reaktion der Edukte zu Produkten bis zum Erreichen eines Gleichgewichtszustandes, Einbringen eines Sorptionsmittels in den Reaktor, Sorption der Produkte durch das Sorptionsmittel, das Verfahren zeichnet sich dadurch aus, dass das mit Produkten beladene Sorptionsmittel in einer Sammelzone im Reaktor gesammelt wird und dass das Katalysatormaterial so im Reaktor angeordnet ist, dass es von der Sammelzone getrennt wird.

Aufgabe der Erfindung ist es daher, ein Verfahren und eine Vorrichtung anzugeben, welche den Umsatz der Reaktion von Wasserstoff und Kohlenstoffdioxid zu Methanol und Wasser erhöhen und dabei energieeffizient sind.

Die Aufgabe wird mit einem Verfahren gemäß Anspruch 1 und einer Vorrichtung gemäß Anspruch 13 gelöst.

Das Verfahren zum Umsetzen von Kohlenstoffdioxid und Wasserstoff zu einem ersten Produkt Methanol und einem zweiten Produkt Wasser umfasst zunächst das Bereitstellen eines Reaktors mit einem Katalysator. In den Reaktor werden Kohlenstoffdioxid und Wasserstoff geführt. In dem Reaktor herrschen dabei ein erster Druck und eine erste Temperatur. In den Reaktor wird weiterhin eine erste flüssige Komponente als Kühlmittel und als Entzugsmittel für das erste Produkt Methanol hinzugeführt. Die erste flüssige Komponente weist dabei eine zweite Temperatur auf, die niedriger als die erste Temperatur ist. Die erste flüssige Komponente ist daher geeignet, den Reaktor zu kühlen. In dem Reaktor erfolgt das Umsetzen des Kohlenstoffdioxids und des Wasserstoffs zu dem ersten Produkt Methanol und dem zweiten Produkt Wasser, wobei die erste Temperatur und der erste Druck in dem Reaktor derart gewählt werden, dass das Methanol wenigstens teilweise gasförmig ist und die erste Komponente und das zweite Produkt Wasser wenigstens teilweise flüssig sind. Das erste gasförmige Produkt Methanol kann dann mittels der wenigstens einen flüssigen Phase, umfassend die erste Komponente und das zweite Produkt Wasser, aus der Gasphase entzogen werden. Ein erster flüssiger Stoffstrom, umfassend die erste Komponente, Methanol und das zweite Produkt Wasser, wird aus dem Reaktor hinausgeführt.

Die erfindungsgemäße Vorrichtung zum Durchführen des Verfahrens zum Umsetzen von Kohlenstoffdioxid und Wasserstoff zu einem ersten Produkt Methanol und einem zweiten Produkt Wasser umfasst einen Reaktor mit einem Katalysator. Die Vorrichtung umfasst weiterhin einen ersten Einlass in den Reaktor zum Zuführen des Kohlenstoffdioxids und des Wasserstoffs in den Reaktor. Die Vorrichtung umfasst weiterhin einen zweiten Einlass in den Reaktor zum Zuführen der ersten flüssigen Komponente. Die Vorrichtung umfasst auch einen ersten Auslass aus dem Reaktor zum Hinausführen eines ersten Stoffstroms umfassend die erste Komponente, Methanol und das zweite Produkt Wasser. Die erfindungsgemäße Vorrichtung umfasst auch ein Vorlagegefäß, gefüllt mit der ersten flüssigen Komponente, welches über eine erste Rohrleitung mit dem Reaktor verbunden ist. Die Reaktionsvorrichtung umfasst neben dem Reaktor auch eine thermische Trennvorrichtung, die mit dem ersten Vorlagegefäß und/oder mit dem Reaktor über eine zweite wasserdurchströmte Rohrleitung verbunden ist.

Als gasförmiges Methanol wird hierbei sowohl Methanol bezeichnet, welches dampfförmig, also kondensierbar, vorliegt als auch Methanol, welches überkritisch vorliegt. Das Entziehen des Methanols aus der Gasphase erfolgt dabei im Falle, dass das Methanol dampfförmig vorliegt mittels Kondensierens. Liegt das Methanol hingegen überkritisch vor, so erfolgt das Entziehen aus der Gasphase mittels Absorption in die erste flüssige Komponente und/oder das zweite Produkt Wasser.

In dem Reaktor stellt sich ein thermodynamisches Gleichgewicht zwischen der Gasphase und der flüssigen Phase ein. Das bedeutet, dass in der Gasphase das zweite Produkt Wasser und die erste Komponente aufgrund des thermodynamischen Gleichgewichts in geringen Mengen vorliegt.

Durch kontinuierlichen Entzug der flüssigen Phase, welche Produkte enthält, aus dem Reaktor wird das Reaktionsgleichgewicht vorteilhaft auf die Seite der Produkte verschoben, so dass der Umsatz gesteigert wird. Der Umsatzgrad des Kohlenstoffdioxids und Wasserstoffs zu Methanol wird mit diesem Verfahren vorteilhaft deutlich erhöht. Es ist mit dem erfindungsgemäßen Verfahren sogar möglich, nahezu Vollumsatz zu erreichen.

Es ist dann vorteilhaft nicht mehr nötig die Gasphase im Kreis zu führen, sodass es ebenso vorteilhaft nicht mehr nötig ist die Gasphase zu komprimieren. Dies führt vorteilhaft dazu, dass das Verfahren energieeffizient ist und dabei einen höheren Umsatz erreicht.

Weiterhin weist die Reaktion von Wasserstoff und Kohlenstoffdioxid zu Methanol und Wasser eine hohe Reaktionsenthalpie auf. Es handelt sich dabei um eine stark exotherme Reaktion, die eine starke Wärmefreisetzung zur Folge hat. Liegt im Eduktgemisch neben Kohlenstoffdioxid auch ein typischerweise geringer Anteil von Kohlenstoffmonoxid vor, so vergrößert sich die Exothermie der Reaktion zusätzlich. Durch den erhöhten Umsatz steigt bei der Reaktion die frei werdende Wärmemenge weiter an. Das Kondensieren des Methanols führt zu einer weiteren Wärmefreisetzung. Das Kühlen dieses Prozesses ist also notwendig. Durch die Auswahl der zweiten Temperatur der ersten flüssigen Komponente, nämlich einer geringeren zweiten Temperatur im Vergleich zur ersten Temperatur, ist es möglich diese Reaktion zu kühlen und eine hohe Wärmefreisetzung vorteilhaft zu verhindern. Weiterhin führt das Kühlen der Reaktion vorteilhaft dazu, dass der Katalysator, welcher typischerweise bei hohen Temperaturen zu einer Deaktivierung neigt, eine längere Lebensdauer aufweist.

Das Zugeben der ersten Komponente mit einer zweiten Temperatur unterhalb der ersten Temperatur führt vorteilhaft weiterhin dazu, dass die Kondensation des Methanols bereits bei geringeren Drücken erfolgt. Vorteilhaft wird die Energieeffizienz somit auch dadurch gesteigert, dass im Reaktor niedrigere Drücke bei erhöhtem Umsatz im Vergleich zu den konventionellen Reaktionsbedingungen herrschen.

Typischerweise werden für die Methanolsynthese Katalysatoren auf Basis von Cu-ZnO-Al₂O₃ oder Cr₂O₃ mit verschiedenen Additiven und Promotern verwendet. Im aus dem Stand der Technik bekannten Hochdruckprozess zur Methanolherstellung werden Katalysatoren auf Basis von ZnO-Cr₂O₃ (ohne Kupfer) verwendet. Derzeit werden neue Katalysatoren basierend auf Gold Au, Silber Ag, Palladium Pd oder Platin Pt entwickelt, sind aber noch nicht kommerziell einsetzbar, da sie bei nur geringen Verbesserungen der Ausbeute teuer sind.

In einer vorteilhaften Ausgestaltung und Weiterbildung der Erfindung wird der erste Stoffstrom in eine thermische Trennvorrichtung geführt und das erste Produkt Methanol in der thermischen Trennvorrichtung von dem zweiten Produkt Wasser getrennt. Vorteilhaft wird so Methanol gewonnen und kann als Edukt für weitere Reaktionen verwendet werden. Insbesondere kann es zu Dimethylether oder Oxymethylenether umgesetzt werden, wobei diese beiden Substanzen als Kraftstoff eingesetzt werden. Das Methanol kann insbesondere auch direkt als Kraftstoff oder zum Beimischen zu Kraftstoff, insbesondere zu Diesel, verwendet werden.

In einer weiteren vorteilhaften Ausgestaltung und Weiterbildung der Erfindung ist die erste flüssige Komponente Wasser. Dieses Wasser mischt sich mit dem zweiten Produkt Wasser und fungiert als Entzugsmittel für das Methanol aus der Gasphase. Das zugeführte Wasser dient dabei auch als Kühlmittel für die Reaktion. Besonders vorteilhaft ist es, wenn in der thermischen Trennvorrichtung das Wasser von dem Methanol getrennt wird. Dann ist es vorteilhaft möglich, das abgetrennte Wasser zurück in den Reaktor als erste flüssige Komponente zu führen. Vorteilhaft wird das Wasser in einem Vorlagebehälter gespeichert, wobei der Vorlagebehälter mit dem Reaktor über eine Rohrleitung verbunden ist, sodass die erste flüssige Komponente von dem Vorlagebehälter in den Reaktor geführt werden kann. Das in der thermischen Trennvorrichtung abgetrennte Wasser kann dann entweder direkt in den Reaktor zurückgeführt werden oder zurück in das Vorlagegefäß geführt werden.

Besonders vorteilhaft liegt das Masseverhältnis von Wasser, welches als erste Komponente hinzu geführt wird, zu Methanol, dem ersten Produkt, bei drei zu eins. Das bedeutet, wenn insbesondere 3 kg Wasser in den Reaktor geführt werden, kann 1 kg Methanol hergestellt und in die flüssige Phase entzogen werden, wobei dieser Prozess nahezu isotherm ist. Vorteilhaft ist somit keine zusätzliche Kühlung des Reaktors notwendig.

Vorteilhaft nimmt die Kapazität des Wassers zur Wärmeaufnahme bei konstanter zweiter Temperatur, also der Eingangstemperatur der ersten flüssigen Komponente in den Reaktor, mit steigender Reaktionstemperatur zu, sodass die benötigte Wassermenge, die benötigt wird, um 1 kg Methanol aus der Gasphase zu entziehen, abnimmt.

In einer weiteren vorteilhaften Ausgestaltung und Weiterbildung der Erfindung ist die erste flüssige Komponente ein Kraftstoff. Als Kraftstoff wird insbesondere Benzin, Diesel oder ein Alkohol verwendet. Vorteilhafterweise stellen diese Kraftstoffe selbst einen Anteil eines möglichen Endprodukts dar. Ein Endprodukt kann insbesondere ein mit Methanol angereicherter Kraftstoff sein. Das Endprodukt Kraftstoff enthält dabei einen definierten Anteil Methanol. Der Kraftstoff wird in dieser Ausführung als erste flüssige Komponente in den Reaktor geführt und dient dabei sowohl als Kühlmittel als auch Entzugsmittel für das Methanol. Im Falle, dass das Methanol überkritisch vorliegt, wird es in die flüssige Phase, welche überwiegend Kraftstoff umfasst, absorbiert.

In der flüssigen Phase befindet sich auch das zweite Produkt Wasser. Dieses ist in technischen Anwendungen häufig unerwünscht. Um das Wasser abzutrennen, kann dann insbesondere eine Destillation des flüssigen Stoffstroms erfolgen. Ist der Kraftstoff vollständig mit Methanol mischbar, jedoch nicht mit dem zweiten Produkt Wasser, bildet sich insbesondere nach dem Hinausführen aus dem Reaktor, insbesondere bei einer weiteren Temperaturerniedrigung außerhalb des Reaktors im Vergleich zu dem Reaktorinneren, ein Zwei-Phasengemisch. Eine Phase beinhaltet dabei im wesentlichen Wasser und Methanol. Die andere Phase umfasst im wesentlichen Methanol und den Kraftstoff. Wird als Kraftstoff ein Alkohol verwendet, sind dies besonders vorteilhaft Ethanol, Butanol oder Hexanol.

Besonders vorteilhaft ist es, wenn der Kraftstoff bei einer erhöhten Temperatur, insbesondere der Innentemperatur des Reaktors, also der ersten Temperatur, insbesondere von wenigstens 100 °C, eine gute Mischbarkeit mit Methanol und Wasser aufweist. Dies führt zu einer guten Aufnahme von Methanol und dem zweiten Produkt Wasser unter Reaktionsbedingungen in dem Reaktor. Wird der erste flüssige Stoffstrom, welcher dann den Kraftstoff, das Methanol, und das zweite Produkt Wasser umfasst, aus dem Reaktor hinaus geführt und auf Temperaturen unterhalb von 100 °C gekühlt, bildet sich aufgrund einer Mischungslücke ein Zweiphasen-Gemisch aus. Besonders vorteilhaft bildet sich dabei eine Wasserphase aus, die nur einen sehr geringen Anteil an Methanol aufweist. Insbesondere weisen längerkettige Alkohole, insbesondere Butanol oder Hexanol geeignete Eigenschaften auf, um das Methanol zwischen den beiden flüssigen Phasen optimal, d.h. insbesondere größtenteils in der kraftstoffreichen Phase, zu verteilen. In diesem Fall kann eine destillative Aufarbeitung des Endprodukts Kraftstoff vorteilhaft mit weniger Trennstufen erfolgen oder es kann auf die destillative Aufarbeitung in Gänze verzichtet werden.

In einer weiteren vorteilhaften Weiterbildung und Ausgestaltung der Erfindung liegt das Massenverhältnis der ersten flüssigen Komponente zu dem Methanol in einem Bereich zwischen 2:1 und 5:1. Vorteilhaft reichen diese Mengen an flüssiger Komponente aus, um die im Reaktor aufgrund der Kondensation und Reaktion entstehende Wärme, aus dem Reaktor zu leiten, sodass der Reaktor nahezu isotherm betrieben wird. Vorteilhaft wird somit der Einsatz einer teuren externen Kühleinheit vermieden und die Energieeffizienz somit erhöht.

In einer weiteren vorteilhaften Ausgestaltung und Weiterbildung der Erfindung liegt der erste Druck in einem Bereich von 100 bar bis 250 bar. Besonders vorteilhaft ist es, wenn der erste Druck möglichst gering innerhalb dieses Bereiches ist. Dies erhöht vorteilhaft die Energieeffizienz des Verfahrens. Allerdings muss der Druck auch ausreichend hoch gewählt werden, um das Umsetzen des Kohlenstoffdioxids zu dem ersten Produkt Methanol mit ausreichend großem Umsatz zu ermöglichen. Als ausreichend großer Umsatz wird ein Umsatz in einem Bereich von 50 % bis 80 % pro Umsatz angesehen. Vorteilhaft kann mit diesem Verfahren nahezu Vollumsatz erreicht werden, wodurch vorteilhaft auf einen Recyclekompressor, welcher zum Komprimieren nicht umgesetzter Edukte vor einem Zurückführen in die Reaktionsvorrichtung angeordnet ist, verzichten werden.

In einer weiteren vorteilhaften Ausgestaltung und Weiterbildung der Erfindung liegt die erste Temperatur in einem Bereich zwischen 200 °C und 300 °C. Die kritische Temperatur von Methanol beträgt 240 °C. Die kritische Temperatur des Wassers liegt bei 374 °C. Demnach liegt Wasser in dem genannten Temperaturbereich dampfförmig vor, sodass das zweite Produkt Wasser vorteilhaft kondensieren kann. Es dient dann als zusätzliches Entzugsmittel des Methanols aus der Gasphase.

In einer weiteren vorteilhaften Ausgestaltung und Weiterbildung der Erfindung liegt die zweite Temperatur der ersten flüssigen Komponente unterhalb der Reaktionstemperatur, insbesondere in einem Bereich zwischen 5 °C und 100 °C. In diesem Temperaturbereich ist es insbesondere möglich, dass die erste flüssige Komponente die im Reaktor entstehende Wärme vollständig aus dem Reaktor herausführen kann. Vorteilhaft ist so eine isotherme Reaktionsführung möglich, ohne eine externe zusätzliche Kühlung zu betreiben.

Weitere Merkmale, Eigenschaften und Vorteile der vorliegenden Erfindung ergeben sich aus der nachfolgenden Beschreibung unter Bezugnahme auf die beiliegenden Figuren. Darin zeigen schematisch:
- Figur 1: eine Vorrichtung mit einem Reaktor zum Umsetzen von Kohlenstoffdioxid und Wasserstoff zu Methanol und Wasser unter Zuführen von Wasser als erste Komponente und einer thermischen Trennvorrichtung;
- Figur 2: ein Diagramm der Ausbeute an Methanol in Abhängigkeit vom Druck für unterschiedliche erste Temperaturen mit und ohne Wasser;
- Figur 3: eine Vorrichtung mit einem Reaktor zum Umsetzen von Kohlenstoffdioxid und Wasserstoff zu Methanol und Wasser unter Zuführen von Ethanol als erste Komponente und einen Phasenabscheider.

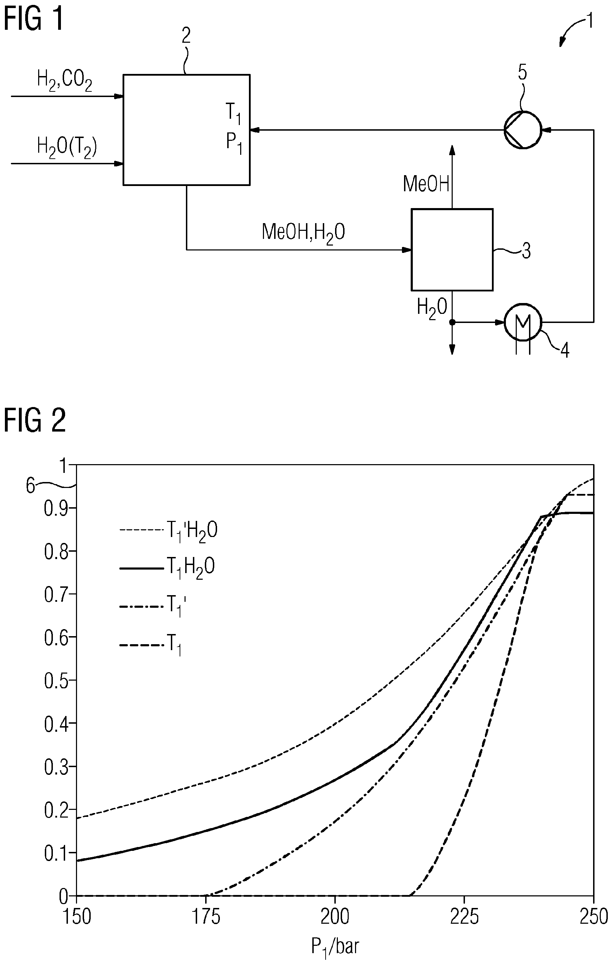

Figur 1 zeigt eine Vorrichtung 1 mit einem Reaktor 2 zum Umsetzen von Kohlenstoffdioxid CO2 und Wasserstoff H2 zu Methanol MeOH und Wasser H2O. Die Vorrichtung 1 umfasst auch eine thermische Trennvorrichtung 3.

Zunächst wird ein Eduktgemisch umfassend Wasserstoff H2 und Kohlenstoffdioxid CO2 in den Reaktor 2 geführt. In dem Eduktgemisch aus Wasserstoff H2 und Kohlenstoffdioxid CO2 kann auch ein geringer Anteil Kohlenstoffmonoxid enthalten sein. Im Vergleich zur üblichen Methanolsynthese aus Kohlenstoffmonoxid ist dieser Anteil aber deutlich geringer. Während der Reaktion entsteht jedoch aus Kohlenstoffdioxid CO2 und Wasserstoff H2 über die Wassergas-Shift-Reaktion ein Teil Kohlenmonoxid CO und Wasser H2O. Typischerweise liegt der Anteil des Kohlenstoffmonoxids im unteren einstelligen Prozentbereich von weniger als 5 %, abhängig vom Katalysator.

In dem Reaktor herrscht eine erste Temperatur T1 und ein erster Druck P1. Die erste Temperatur T1 ist in diesem Beispiel 250 °C und der erste Druck ist in diesem Beispiel 240 bar. Typischerweise werden H2 und CO2 in einem Kompressor (nicht in Figur 1 gezeigt), welcher vor dem Reaktor 2 angeordnet ist, komprimiert. In dem Reaktor 2 befindet sich weiterhin ein Katalysator, der die Reaktion von Kohlenstoffdioxid mit Wasserstoff zu Methanol und Wasser katalysiert.

Typischerweise werden für die Methanolsynthese Katalysatoren auf Basis von Cu-ZnO-Al₂O₃ oder Cr₂O₃ mit verschiedenen Additiven und Promotern verwendet. Derzeit werden neue Katalysatoren basierend auf Gold Au, Silber Ag, Palladium Pd oder Platin Pt entwickelt, sind aber noch nicht kommerziell einsetzbar, da sie bei nur geringen Verbesserungen der Ausbeute teuer sind.

Als erste flüssige Komponente K 1 wird in diesem Beispiel Wasser H2O eingesetzt. Das Wasser wird dem Reaktor 2 zugeführt. Das Wasser kann insbesondere auch im Reaktor versprüht werden. Besonders vorteilhaft wird das Wasser in der Nähe des Katalysators hinzu geführt, um diesen zu kühlen. Die zweite Temperatur T2 des hinzugefügten Wassers H2O ist kleiner als die Reaktortemperatur. In diesem Beispiel beträgt sie insbesondere 50 °C. In dem Reaktor wird aus Wasserstoff H2 und Kohlenstoffdioxid CO2 das erste Produkt Methanol MeOH und das zweite Produkt Wasser H2O hergestellt. Das Methanol MeOH absorbiert bei diesen Bedingungen in das flüssige Wasser. Das flüssige Wasser befindet sich typischerweise am Boden des Reaktors. Die flüssige Mischung aus Wasser H2O, welches aus der Reaktion als zweites Produkt und als zugeführtes Wasser als erste flüssige Komponente stammt, und das Methanol MeOH werden als flüssiger Stoffstrom aus dem Reaktor hinaus geführt. Der flüssige Stoffstrom wird in eine thermische Trennvorrichtung 3 geführt.

In diesem Beispiel ist die thermische Trennvorrichtung eine Destillationsvorrichtung. Es sind aber ebenso weitere thermische Trennverfahren wie insbesondere Pervaporation, adsorptive Verfahren oder weitere Membranverfahren einsetzbar. In der Destillationsvorrichtung 3 werden Methanol MeOH und Wasser H2O voneinander getrennt. Das Wasser H2O wird wenigstens teilweise zurück in den Reaktor 2 geführt. Das Wasser wird in einem ersten Kondensator 4 abgekühlt. Das abgekühlte Wasser wird mittels einer Umwälzpumpe 5 zurück in den Reaktor befördert. Alternativ oder zusätzlich kann das Wasser in ein Vorratsgefäß geführt werden, indem sich das frische Wasser vor der Reaktion befindet, und anschließend zusammen mit der ersten flüssigen Komponente Wasser in den Reaktor geführt werden.

Figur 2 zeigt ein Diagramm, bei welchem die Ausbeute an Methanol in der Flüssigphase 6 gegen den ersten Druck P1 aufgetragen ist. Die Ausbeute ist hierbei definiert als der Molenstrom an Methanol in der Flüssigphase 6 bezogen auf den Molenstrom Kohlenstoffdioxid in den Reaktor 2. Es ist die Ausbeute in Abhängigkeit des Drucks P1 für zwei unterschiedliche Temperaturen, eine erste Temperatur T1 von 250 °C und eine erste Temperatur T1' von 225 °C aufgetragen. Die Reaktion findet hier in der Gasphase statt. Das Diagramm zeigt, dass bei niedrigen Drücken die Ausbeute nahezu bei 0 liegt. Erst bei Drücken ab 175 bar bzw. 200 bar beginnt die Ausbeute 6 zu steigen.

Weiterhin ist die Ausbeute bei der ersten Temperatur T1 und der ersten Temperatur T1' unter Zuführen von Wasser bei einer zweiten Temperatur von 50 °C als erste Komponente aufgezeigt. Es wird die doppelte Molmenge an Wasser im Vergleich zu Wasserstoff zu dem Reaktor 2 hinzugegeben. Es wird deutlich, dass für den Fall, dass Wasser als erste flüssige Komponente in den Reaktor geführt wird, die Ausbeute bereits bei niedrigeren Drücken deutlich steigt. Dies verdeutlicht, dass bereits ohne dass der flüssige Strom aus dem Reaktor geführt wird, die Ausbeute durch das Hinzuführen von Wasser steigt, da das erste Produkt Methanol MeOH in dem Wasser absorbiert wird. Durch das kontinuierliche Hinausführen der Methanol-Wassermischung wird das Produkt kontinuierlich aus der Gasphase entzogen und dann aus dem Reaktor geführt. Das Reaktionsgleichgewicht wird daher auf die Seite der Produkte verschoben, was die Ausbeute erhöht. Zusätzlich dazu wird durch das Hinausführend der Produkte aus dem Reaktor, das Reaktionsgleichgewicht bei konstanter Ausbeute zu niedrigeren Drücken hin verschoben. Demnach kann für den Reaktor 2 eine niedrigere erste Temperatur T1 und ein niedrigerer erster Druck P1 ausgewählt werden als ohne das Zuführen des Wassers, wobei sogar vorteilhaft die Ausbeute erhöht wird.

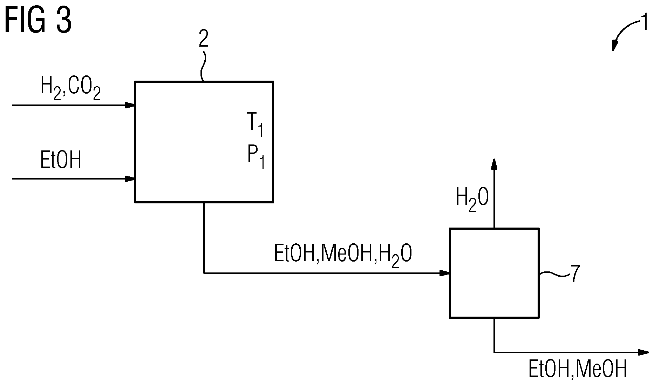

Figur 3 zeigt ein zweites Ausführungsbeispiel, bei welchem in die Vorrichtung 1 als erste Komponente Ethanol in den Reaktor 2 geführt wird. Ebenso wie beim ersten Ausführungsbeispiel wird dem Reaktor 2 Wasserstoff H2 und Kohlenstoffdioxid CO2 hinzugeführt. In dem Reaktor herrschen ein erster Druck P1 und eine erste Temperatur T1. In der Gasphase reagieren Wasserstoff H2 und Kohlenstoffdioxid CO2 zu Methanol MeOH und Wasser H2O. Das Methanol MeOH absorbiert in dem Ethanol EtOH und das zweite Produkt Wasser H2O kondensiert. Aus dem Reaktor 2 wird der erste Stoffstrom umfassend Ethanol EtOH, Methanol MeOH und Wasser H2O kontinuierlich hinausgeführt. Dadurch wird die Reaktion auf die Produktseite verschoben, was die Ausbeute an Methanol MeOH deutlich erhöht. Das Endprodukt ist in diesem Beispiel ein Kraftstoff, welcher Ethanol und Methanol umfasst, aber kein Wasser.

Daher muss das Wasser in der Trennvorrichtung 7 abgetrennt werden. Die Trennvorrichtung 7 ist ebenso wie beim ersten Ausführungsbeispiel eine Destillationsvorrichtung. Für den Fall, dass als erste flüssige Komponente 3 längerkettiger Alkohol verwendet wird, ist es möglich, die Destillationsvorrichtung 7 durch einen Phasenabscheider zu ersetzen. Durch Einstellen einer niedrigeren Temperatur kann dann ausgenutzt werden, dass sich der längerkettige Alkohol und das Methanol MeOH als organische Phase von dem Wasser trennen, sodass zwei flüssige Phasen vorliegen. Verteilt sich das Methanol MeOH zwischen dem Alkohol EtOH und dem Wasser H2O so, dass er überwiegend in der Alkoholphase vorliegt, kann dann die alkoholhaltige Phase in dem Phasenabscheider abgetrennt werden. Vorteilhaft wird so der energetische Aufwand der Trenndung verringert, wodurch weniger Trennstufen in der Destillationskolonne benötigt werden und somit die Investitionskosten sinken. Besonders vorteilhaft ist es sogar möglich, den Einsatz eines Destillationsverfahrens zu vermeiden.

Das Verhältnis von zugeführtem Ethanol zu kondensiertem Methanol in der Flüssigphase liegt bei 4 zu 1. Es muss also 4 kg Ethanol eingesetzt werden, um 1 kg Methanol in der Flüssigphase zu erhalten. Vorteilhaft ist es bei diesem Verhältnis auch möglich, die in der Reaktion entstehende Wärme abzuführen. Vorteilhaft wird so eine zusätzliche Kühlvorrichtung in dem Reaktor 2 vermieden. Insbesondere ist es vorteilhaft das Ethanol oder, im Falle des ersten Ausführungsbeispiel, das Wasser, in der Nähe des Katalysators zu versprühen, um dort direkt die entstehende Reaktionswärme abzuführen.

## Patentansprüche

1. Verfahren zum Umsetzen eines Eduktgemischs umfassend Kohlenstoffdioxid (CO2) und Wasserstoff (H2) zu einem ersten Produkt Methanol (MeOH) und einem zweiten Produkt Wasser (H₂O) mit folgenden Schritten:
- Bereitstellen eines Reaktors (2) mit einem Katalysator,
- Zuführen von Kohlenstoffdioxid (CO2) und Wasserstoff (H2) in den Reaktor (2), wobei in dem Reaktor (2) ein erster Druck (P1) und eine erste Temperatur (T1) herrschen,
- Zuführen einer ersten flüssigen Komponente (K1), die ein Kühl- und Entzugsmittel zum Entziehen des ersten Produkts Methanol (MeOH) ist, in den Reaktor (2), wobei die erste flüssige Komponente (K1) eine zweite Temperatur (T2) aufweist, die niedriger als die erste Temperatur (T1) ist,
- Umsetzen des Kohlenstoffdioxids (CO2) und des Wasserstoffs (H2) zu dem ersten Produkt Methanol (MeOH)und dem zweiten Produkt Wasser (H2O), wobei die erste Temperatur (T1) und der erste Druck (P1) in dem Reaktor (2) derart gewählt werden, dass das Methanol (MeOH) gasförmig ist und die erste Komponente (K1) und das zweite Produkt Wasser (H2O) flüssig sind,
- Entziehen des ersten gasförmigen Produkts Methanol (MeOH) aus einer Gasphase in wenigstens eine flüssige Phase umfassend die erste Komponente (K1) und das zweite Produkt Wasser (H₂O),
- Hinausführen eines ersten flüssigen Stoffstroms umfassend die erste Komponente (K1), das erste Produkt Methanol (MeOH) und das zweite Produkt Wasser (H2O) aus dem Reaktor (2).

2. Verfahren nach Anspruch 1, wobei das Entziehen als Kondensieren und/oder Absorbieren erfolgt.

3. Verfahren nach Anspruch 1 oder 2, wobei der erste Stoffstrom in eine Trennvorrichtung (3) geführt wird und das erste Produkt Methanol (MeOH) in der Trennvorrichtung (3) abgetrennt wird.

4. Verfahren nach einem der Ansprüche 1, 2 oder 3, wobei die erste flüssige Komponente (K1) Wasser ist.

5. Verfahren nach einem der vorhergehenden Ansprüche, wobei die erste flüssige Komponente (K1) Wasser und das zweite Produkt Wasser (H₂O) in der Trennvorrichtung (3) abgetrennt werden und das Wasser als die erste flüssige Komponente (K1) in den Reaktor (2) zurückgeführt wird.

6. Verfahren nach einem der Ansprüche 1, 2 oder 3, wobei die erste Komponente (K1) ein Kraftstoff ist.

7. Verfahren nach Anspruch 6, wobei der Kraftstoff Benzin, Diesel oder ein Alkohol ist.

8. Verfahren nach einem der vorhergehenden Ansprüche, wobei das Massenverhältnis der ersten Komponente (K1) zu Methanol (MeOH) in einem Bereich von 2:1 bis 5:1 liegt.

9. Verfahren nach Anspruch 4 oder 5, wobei das Massenverhältnis der ersten Komponente (K1) Wasser zu Methanol (MeOH) 3:1 beträgt.

10. Verfahren nach einem der vorhergehenden Ansprüche, wobei der erste Druck (P1) in einem Bereich zwischen 100 bar und 300 bar liegt.

11. Verfahren nach einem der vorhergehenden Ansprüche, wobei die erste Temperatur (T1) in einem Bereich zwischen 200° C und 300° C liegt.

12. Verfahren nach einem der vorhergehenden Ansprüche, wobei die zweite Temperatur (T2) in einem Bereich von 5° C bis 100° C liegt.

13. Reaktionsvorrichtung (1) zum Durchführen eines Verfahrens nach einem der Ansprüche 1 bis 12 umfassend:
- einen Reaktor (2) zum Umsetzen von Kohlenstoffdioxid (CO2) und Wasserstoff (H2) zu einem ersten Produkt Wasser (H₂O) und einem zweiten Produkt Methanol (MeOH),
- einen ersten Einlass zum Zuführen des Kohlenstoffdioxids (CO2) und des Wasserstoffs (H2) in den Reaktor (2),
- einen zweiten Einlass zum Zuführen einer ersten flüssigen Komponente (K1) in den Reaktor (2),
- einen ersten Auslass zum Hinausführen eines ersten flüssigen Stoffstroms umfassend das erste und das zweite Produkt (MeOH, H2O) und die erste Komponente (K1),
- ein erstes Vorlagegefäß mit der ersten flüssigen Komponente (K1), welches über eine erste Rohrleitung mit dem zweiten Einlass verbunden ist,
- eine thermische Trennvorrichtung (3), die mit dem ersten Vorlagegefäß und/oder mit dem Reaktor (2) über eine zweite wasserdurchströmte Rohrleitung verbunden ist.

## Claims

1. Process for converting a starting material mixture comprising carbon dioxide (CO₂) and hydrogen (H₂) into a first product, methanol (MeOH), and a second product, water (H₂O), having the following steps:
- providing a reactor (2) containing a catalyst,
- feeding carbon dioxide (CO₂) and hydrogen (H₂) into the reactor (2), wherein the reactor (2) is at a first pressure (P1) and a first temperature (T1),
- feeding a first liquid component (C1) as coolant and as an agent for removing the first product, methanol (MeOH), into the reactor (2), wherein the first liquid component (C1) is at a second temperature (T2) that is lower than the first temperature (T1),
- converting the carbon dioxide (CO₂) and hydrogen (H₂) into the first product, methanol (MeOH), and the second product, water (H₂O), wherein the first temperature (T1) and the first pressure (P1) in the reactor (2) are set so that the methanol (MeOH) is in the gaseous state and the first component (C1) and the second product, water (H₂O), are liquid,
- removing the first gaseous product, methanol (MeOH), from a gas phase in at least one liquid phase comprising the first component (C1) and the second product, water (H₂O),
- discharging a first liquid material stream comprising the first component (C1), the first product, methanol (MeOH), and the second product, water (H₂O), from the reactor (2).

2. Process according to Claim 1, wherein removal takes place through condensation and/or absorption.

3. Process according to Claim 1 or 2, wherein the first material stream is fed into a separator (3) and the first product, methanol (MeOH), is separated in the separator (3).

4. Process according to any of Claims 1, 2 or 3, wherein the first liquid component (C1) is water.

5. Process according to any of the preceding claims, wherein the first liquid component (C1), water, and the second product, water (H₂O), are separated in the separator (3) and the water is fed back into the reactor (2) as the first liquid component (C1) .

6. Process according to any of Claims 1, 2 or 3, wherein the first component (C1) is a fuel.

7. Process according to Claim 6, wherein the fuel is gasoline, diesel or an alcohol.

8. Process according to any of the preceding claims, wherein the mass ratio of the first component (C1) to methanol (MeOH) is in a range from 2:1 to 5:1.

9. Process according to Claim 4 or 5, wherein the mass ratio of the first component (C1), water, to methanol (MeOH) is 3:1.

10. Process according to any of the preceding claims, wherein the first pressure (P1) is in a range between 100 bar and 300 bar.

11. Process according to any of the preceding claims, wherein the first temperature (T1) is in a range between 200°C and 300°C.

12. Process according to any of the preceding claims, wherein the second temperature (T2) is in a range between 5°C and 100°C.

13. Reaction apparatus (1) for executing a process according to any of Claims 1 to 12 comprising:
- a reactor (2) for converting carbon dioxide (CO₂) and hydrogen (H₂) into a first product, water (H₂O), and a second product, methanol (MeOH),
- a first inlet for feeding the carbon dioxide (CO₂) and the hydrogen (H₂) into the reactor (2),
- a second inlet for feeding a first liquid component (C1) into the reactor (2),
- a first outlet for discharging a first liquid material stream comprising the first and second product (MeOH, H₂O) and the first component (C1),
- a first holding tank containing the first liquid component (C1), which is connected to the second inlet through a first pipeline,
- a thermal separator (3) which is connected to the first holding tank and/or to the reactor (2) through a second pipeline carrying water.

## Revendications

1. Procédé de transformation d'un mélange éduit comprenant du dioxyde de carbone (CO2) et de l'hydrogène (H2) en un premier produit du méthanol (MeOH), et en un deuxième produit, de l'eau (H2O), comprenant les stades suivants :
- on se procure un réacteur (2) avec un catalyseur,
- on envoie du dioxyde de carbone (CO2) et de l'hydrogène (H2) dans le réacteur (2), une première pression (P1) et une première température (T1) régnant dans le réacteur (2),
- on envoie dans le réacteur (2) un premier composant (K1) liquide, qui est un agent de refroidissement et d'extraction pour extraire le premier produit, du méthanol (MeOH), le premier composant (K1) liquide ayant une deuxième température (T2) qui est plus basse que la première température (T1),
- on transforme le dioxyde de carbone (CO2) et l'hydrogène (H2) en le premier produit, du méthanol (MeOH), et en le deuxième produit, de l'eau (H2O), la première température (T1) et la première pression (P1) dans le réacteur (2) étant choisies de manière à ce que le méthanol (MeOH) soit sous forme gazeuse et de manière à ce que le premier composant (K1) et le deuxième produit, de l'eau (H2O), soient liquides,
- on extrait le premier produit sous forme gazeuse, du méthanol (MeOH), d'une phase gazeuse dans au moins une phase liquide comprenant le premier composant (K1) et le deuxième produit, de l'eau (H2O),
- on fait sortir du réacteur (2) un premier courant de matière liquide comprenant le premier composant (K1), le premier produit, du méthanol (MeOH), et le deuxième produit, de l'eau (H2O).

2. Procédé suivant la revendication 1, dans lequel on effectue l'extraction par condensation et/ou absorption.

3. Procédé suivant les revendications 1 ou 2, dans lequel on envoie le premier courant de matière à une installation (3) de séparation et on sépare le premier produit, du méthanol (MeOH), dans l'installation (3) de séparation.

4. Procédé suivant l'une des revendications 1, 2 ou 3, dans lequel le premier composant (K1) liquide est de l'eau.

5. Procédé suivant l'une des revendications précédentes, dans lequel on sépare dans l'installation (3) de séparation le premier composant (K1) liquide de l'eau et le deuxième produit, de l'eau (H2O), et on retourne l'eau comme premier composant (K1) liquide au réacteur (2).

6. Procédé suivant l'une des revendications 1, 2 ou 3, dans lequel le premier composant (K1) est un carburant.

7. Procédé suivant la revendication 6, dans lequel le carburant est de l'essence, du carburant diesel ou un alcool.

8. Procédé suivant l'une des revendications précédentes, dans lequel le rapport massique du premier composant (K1) au méthanol (MeOH) est dans une plage 2:1 à 5:1.

9. Procédé suivant la revendication 4 ou 5, dans lequel le rapport massique du premier constituant (K1), de l'eau, au méthanol (MeOH) est de 3:1.

10. Procédé suivant l'une des revendications précédentes, dans lequel la première pression (P1) est dans une plage comprise entre 100 bar et 300 car.

11. Procédé suivant l'une des revendications précédentes, dans lequel la première température (T1) est dans une plage comprise entre 200°C et 300 °C.

12. Procédé suivant l'une des revendications précédentes, dans lequel la deuxième température (T2) est dans une plage de 5°C à 100 °C.

13. Installation (1) de réaction pour effectuer un procédé suivant l'une des revendications 1 à 12, comprenant :
- un réacteur (2) de transformation du dioxyde de carbone (CO2) et de l'eau (H2) en un premier produit, de l'eau (H2O), et en deuxième produit, du méthanol (MeOH),
- une première entrée pour envoyer le dioxyde de carbone (CO2) et l'hydrogène (H2) dans le réacteur (2),
- une deuxième entrée pour envoyer un premier composant (K1) liquide dans le réacteur (2),
- une première sortie pour faire sortir un premier courant de matière liquide comprenant le premier et le deuxième produit (MeOH, H2O) et le premier composant (K1),
- un premier récipient collecteur ayant le premier constituant (K1) liquide, qui communique avec la deuxième entrée par une première canalisation,
- une installation (3) de séparation thermique, qui communique avec le premier récipient collecteur et/ou avec le réacteur (2) par une deuxième canalisation dans laquelle passe de l'eau.
